# FASCICULE DE BREVET EUROPEEN

(11) **EP 2 855 445 B1**
(45) Date de publication et mention de la délivrance du brevet: **20.07.2016**
(21) Numéro de dépôt: 13723888.7
(22) Date de dépôt: 26.04.2013
(51) Int. Cl.: C07D 401/04, H01M 10/052

(54) **SEL D'ANIONS BICYCLIQUES AROMATIQUES POUR BATTERIES LI-ION**
SALZ AUS BICYCLISCHEN AROMATISCHEN ANIONEN FÜR LI-IONEN-BATTERIEN
SALT OF BICYCLIC AROMATIC ANIONS FOR LI-ION BATTERIES

(30) Priorité: 04.06.2012 FR 1255154
(43) Date de publication de la demande: 08.04.2015
(73) Titulaire: Arkema France, 92700 Colombes (FR)
(72) Inventeur: SCHMIDT, Grégory, 69700 Saint Andéol le Château (FR)
(74) Mandataire: Dang, Doris
(86) Numéro de dépôt international: PCT/FR2013/050939
(87) Numéro de publication internationale: WO 2013/182768

(56) Documents cités:
- WO-A1-2010/023413
- US-A1- 2004 009 393

## Description

### DOMAINE DE L'INVENTION

La présente invention concerne les composés imidazole bicycliques, et leurs sels, leurs procédés de fabrication ainsi que leurs utilisations, notamment comme composant d'électrolyte pour batteries.

### ARRIERE-PLAN TECHNIQUE

Une batterie lithium-ion ou sodium-ion comprend au moins une électrode négative, une électrode positive, un séparateur et un électrolyte. L'électrolyte est constitué d'un sel de lithium ou sodium dissous dans un solvant qui est généralement un mélange de carbonates organiques, afin d'avoir un bon compromis entre la viscosité et la constante diélectrique.

Parmi les sels les plus utilisés figure l'hexafluorophosphate de lithium (LiPF₆), qui possède beaucoup des nombreuses qualités requises mais présente le désavantage de se dégrader sous forme de gaz d'acide fluorhydrique. Cela pose des problèmes de sécurité, notamment dans le contexte de l'utilisation prochaine des batteries lithium-ion pour les véhicules particuliers.

Les prérequis pour avoir un sel d'électrolyte sont une bonne dissociation chimique entre le cation et l'anion ce qui implique une charge négative sur l'anion délocalisée ou diminuée par des effets attracteurs.

Des sels basés sur l'effet attracteur ont donc été développés tels que le LiTFSI (bis(trifluoromethanesulfonyl)imidure de lithium) et le LiFSI (bis(fluorosulfonyl)imidure de lithium).

D'autres sels cette fois basés sur la délocalisation de la charge ont également été développés tels que le LiTDI (1-trifluorométhyl-4,5-dicarbonitrile-imidazolate de lithium), ainsi que cela est enseigné dans le document WO 2010/023413. Mais ces derniers présentent des conductivités ioniques plus faibles que ceux précédemment cités.

La demanderesse a découvert que la présence d'un second cycle aromatique permettrait d'augmenter la délocalisation de la charge négative et ainsi augmenter cette conductivité ionique.

### RESUME DE L'INVENTION

Dans ce qui suit, on désigne par
- DAMN : le diaminomaléonitrile et est représenté par la formule (I) 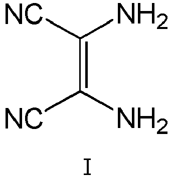
- Les composés (II) sont représentés par les formules générales développées ci-dessous. Ils sont désignés sous (IIa) lorsque le cycle aromatique est à 6 atomes et sous (IIb) pour un cycle aromatique à 5 atomes 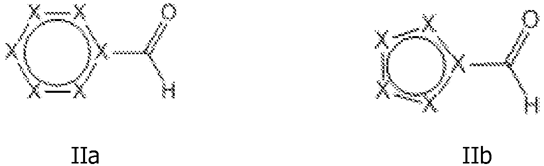
- Les composés (III) sont représentés par les formules générales développées ci-dessous. Ils sont désignés sous (IIIa) lorsque le cycle aromatique est à 6 atomes et sous (IIIb) pour un cycle aromatique à 5 atomes 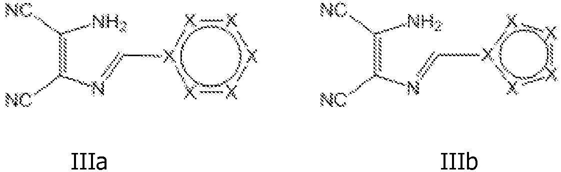
- Les composés imidazole bicycliques (IV) sont représentés par les formules générales développées ci-dessous. Ils sont désignés sous (IVa) lorsque le cycle aromatique est à 6 atomes et sous (IVb) pour un cycle aromatique à 5 atomes 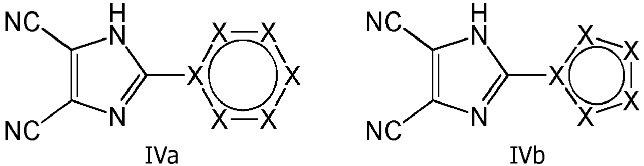
- Les sels des composés imidazole bicycliques (V) sont représentés par les formules générales développées ci-dessous. Ils sont désignés sous (Va) lorsque le cycle aromatique est à 6 atomes et sous (Vb) pour un cycle aromatique à 5 atomes

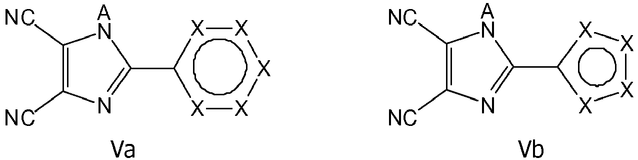

Dans les formules générales ci-dessus, A représente un cation monovalent, X représente indépendamment un atome de carbone, un atome d'oxygène, un atome de soufre, un atome de phosphore ou un atome d'azote.

Lorsque X représente un atome de carbone, de phosphore ou azote, les substituants peuvent être indépendamment des groupements electro-attracteurs ou électro-donneurs définis par un paramètre de Hammett (le paramètre de Hammett est une constante tabulée qui est déterminée pour une série de groupes substituants en mesurant la constante de dissociation des acides benzoïques correspondants) compris entre -0,7 et 1,0. De préférence, les substituants sont choisis parmi un groupement cyano (CN), un groupement R₁, un groupement éther de type OR₁, un groupement amino de type N(R₁)₂, un groupement ester de type CO₂R₁, un groupement sulfonyle type SO₂R₁ ou un groupement phosphonyle type PO₂R₁, où R₁ a pour formule CₙHₘX'ₚ avec n compris entre 0 et 6, m compris entre 0 et 13, X' est un halogéne (F, CI, Br et I) et p compris entre 1 et 13.

L'invention concerne en premier lieu les composés imidazole bicycliques (IV) et leurs sels (V).

L'invention concerne en deuxième lieu les procédés de fabrication de composés imidazole bicycliques (IV) et leurs sels (V).

L'invention concerne en troisième lieu l'utilisation des composés de formule (V).

### DESCRIPTION DETAILLEE DE L'INVENTION

L'invention est maintenant décrite plus en détail et de façon non limitative dans la description qui suit.

Les sels des composés imidazole bicycliques (V), selon la présente invention sont représentés par la formule générale ci-dessus dans laquelle A représente un cation monovalent A, par exemple un métal alcalin.

Le métal alcalin préféré est choisi parmi le lithium et le sodium.

Lorsque X dans la formule générale représente un atome de carbone, de phosphore ou d'azote, les sels (V) peuvent être substitués. Les substituants préférés sont des groupements électro-attracteurs ou électro-donneurs, en particulier ceux ayant un paramètre de Hammett compris entre -0,7 et 1.

Les groupements électro-attracteurs et électro-donneurs particulièrement préférés, sont choisis parmi un groupement cyano (CN), un groupement R₁, un groupement éther de type OR₁, un groupement amino de type N(R₁)₂, un groupement ester de type CO₂R₁, un groupement sulfonyle type SO₂R₁ ou un groupement phosphonyle type PO₂R₁. où R₁ a pour formule CₙHₘX'ₚ avec n compris entre 0 et 6, m compris entre 0 et 13, X' est un halogéne (F, CI, Br et I) et p compris entre 1 et 13.

### Préparation des sels des composés d'imidazole bicycliques (imidazolates bicycliques) et des composés d'imidazole bicycliques

Les imidazolates bicycliques (V) peuvent être préparés à partir des composés imidazole (IV) en faisant réagir celui-ci avec une base AZ, avec A ayant la même signification que ci-dessus et Z représentant un anion hydrure, hydroxyde ou carbonate. De préférence AZ est choisi parmi l'hydrure de lithium, le carbonate de lithium, l'hydroxyde de lithium, l'hydrure de sodium, le carbonate de sodium, l'hydroxyde de sodium et les combinaisons de ceux-ci.

IV + AZ → V + AH

Les composés (IV) peuvent être préparés à partir de la condensation d'un aldéhyde aromatique de formule générale (II) et du DAMN (I).

Le procédé de préparation des composés imidazole bicycliques (IV) comprend (i) une étape réactionnelle du DAMN de formule (I) avec un aldéhyde aromatique de formule générale (II) à une température comprise entre 0 et 80°C, de préférence de 10 à 50°C, plus préférentiellement de 20 à 30°C, en présence éventuellement d'un solvant pour donner un composé de formule (III), suivie (ii) d'une étape de deshydrogénation du composé de formule (III).

DAMN + II → III + H₂O (i)

III - H₂ → IV (ii)

L'étape (i) est de préférence mise en oeuvre en présence d'un solvant. Tout composé permettant de solubiliser le(s) réactif(s) peut être utilisé comme solvant. A titre indicatif, on peut citer le dioxane, l'acétonitrile, ou l'ethanol.

Lorsque l'étape (i) est mise en oeuvre en présence d'un solvant, la concentration du DAMN dans le milieu réactionnel est de préférence de 0,001 à 2 mol/l, plus préférentiellement de 0,1 mol/l à 1 mol/l. Le rapport molaire du composé (I) sur le composé (II) est de préférence de 0,25 à 1,5, plus préférentiellement de 0,5 à 1,25.

La durée de l'étape (i) est de préférence de 1 à 12 heures, plus particulièrement de 1 à 5 heures, par exemple d'environ 2 heures.

De préférence, l'étape (i) est mise en oeuvre en présence d'un catalyseur acide, éventuellement par ajout d'acide sulfurique ou d'un acide carboxylique, tel que l'acide trifluoroacétique, l'acide acétique ou l'acide benzoïque dans le milieu réactionnel.

Selon un mode de réalisation de l'invention, la température de la réaction peut être constante tout au long de la première étape.

Selon un autre mode de réalisation de l'invention, la température est croissante tout au long de l'étape (i).

L'etape (ii) peut être réalisée en présence de composé suceptible de réagir avec l'hydrogène, comme l'oxygène, l'eau oxygénée et les péroxydes, le N-chlorosuccinimide, le N-bromosuccinimide, le chlorure d'hydroxyle, le fluorure d'hydroxyle , les composés à squelette type quinone

A l'issue de cette réaction, le composé imidazole bicyclique de formule (IV) est de préférence isolé et purifié.

Ainsi, le milieu réactionnel peut être évaporé et l'imidazole (III) recristallisé dans de l'eau pour ensuite être récupéré par filtration. Le solide obtenu peut être dissous dans une solution aqueuse de base AZ, de préférence lithiée ou sodée avec une concentration allant de 10⁻⁵ mol/l à la concentration de saturation. Une fois le sel de composé de formule (IV) est formé la solution peut subir plusieurs traitements au charbon actif. La solution peut ensuite être évaporée pour donner le sel de formule (IV).

### Préparation d'un électrolyte

Les composés de formule (V) peuvent être utilisés pour la préparation d'un électrolyte, en les dissolvant dans un solvant approprié.

Le solvant peut être constitué d'au moins un composé choisi parmi les carbonates, les glymes, les nitriles et les sulfones.

Comme carbonate, on peut citer notamment l'éthylene carbonate, le dimethylcarbonate, l'éthylméthylcarbonate, le diéthylcarbonate, le propylene carbonate, carbonate de glycerol.

Comme glymes on peut citer notamment l'éthylène glycol diméthyléther, le diéthylène glycol diméthyléther, le dipropylène glycol diméthyléther, le diéthylène glycol diéthyléther, le triéthylène glycol diméthyléther, le diéthylène glycol dibutyléther, le tétraéthylène glycol diméthyléther et le diéthylène glycol t-buthylméthyléther.

Comme nitriles on peut citer notamment, l'acétonitrile, le propionitrile, le butyronitrile, le méthoxypropionitrile, l'isobutyronitrile et les composés fluorés dérivants des composés précédents.

Comme sulfones on peut notamment citer le diméthylsulfone, le sulfolane, l'éthylméthylsulfone, le propylméthylsulfone, l'isopropylméthylsulfone, l'isopropyléthylsulfone, le tertbutyléthylsulfone, le tertbutylméthylsulfone et le tertbutylpropylsulfone.

Le solvant est de préférence constitué d'un mélange de composés, avantageusement de 2 à 5 choisis parmi les carbonates et/ou glymes et/ou les sulfones précédemment cités.

Les proportions en poids de chacun des composés constituant le solvant sont de préférence comprises entre 1 et 10 par rapport au constituant en plus faible quantité, plus préférentiellement entre 1 et 8.

La concentration en composé de formule (V) dans l'électrolyte est de préférence de 0,1 mol/l à 5 mol/l, plus préférentiellement de 0,2 mol/l à 2,5 mol/l. De préférence, l'électrolyte est constitué d'un mélange d'au moins deux sels de lithium choisi parmi le sel d'imidazolate (IV), le LiPF₆, le LiBF₄, le CF₃COOLi, le CF₃SO₂Li, le LiTFSI (bis(trifluoromethanesulfonyl)imidure de lithium), le LiFSI (bis(fluorosulfonyl)imidure de lithium), le LiTDI (1-trifluorométhyl-4,5-dicarbonitrile-imidazolate de lithium) et le LiPDI (1-pentafluoroéthyl-4,5-dicarbonitrile-imidazolate de lithium). La quantité de chaque sel de lithium présent dans le mélange peut varier dans de larges limites et représente en général, entre 0,1 et 99,9 % en poids par rapport au poids total des sels présents dans le mélange, et de préférence entre 1 et 99 % en poids.

### EXEMPLE

L'exemple suivant illustre l'invention sans la limiter.

On ajoute quelques gouttes d'acide sulfurique, dans 50 ml d'acétonitrile, contenant 1,19 g de DAMN préalablement dissous, et 1,47g de p-CN-benzaldéhyde .Un précipité jaune apparaît alors. La réaction est laissée sous agitation pendant 3 heures. La solution est filtrée et le solide est rincé avec de l'acétonitrile puis de l'éther. Le solide est ensuite séché sous vide à 110°C.

Dans un ballon de 50 ml, 0,94 g de K₂CO₃ puis 0,90 g de N-chlorosuccinimide sont ajoutés à 1,0 g du solide précédemment obtenu dissous dans 25 mL de dimethylformamide. La solution est agitée à température ambiante pendant une nuit. 150 ml d'eau sont ajoutés en fin de réaction. La solution est acidifiée jusqu'à pH = 1 avec de l'acide sulfurique. La phase aqueuse est alors extraite avec 2 fois 100 mL d'acétate d'éthyle. Les phases organiques sont séchées puis évaporées. L'analyse RMN du résidu montre la présence du produit désiré. Le résidu est repris dans de l'eau en présence d'un excès de carbonate de lithium. La solution est agitée à température ambiante pendant 3 heures. La solution est filtrée puis extraite avec 2 fois 250 mL d'éther. La phase aqueuse est ensuite traitée au charbon actif pendant 2 heures à 50°C. La solution est filtrée puis évaporée. Le résidu est ensuite repris dans de l'acétonitrile, la partie insoluble correspondant au carbonate de lithium est éliminée par filtration. Le filtrat est alors évaporé et donne un solide jaune qui est le sel de lithium de formule suivante :

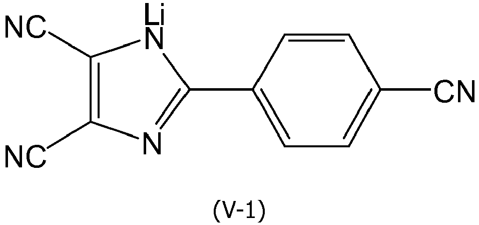

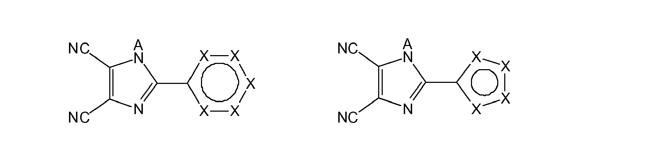

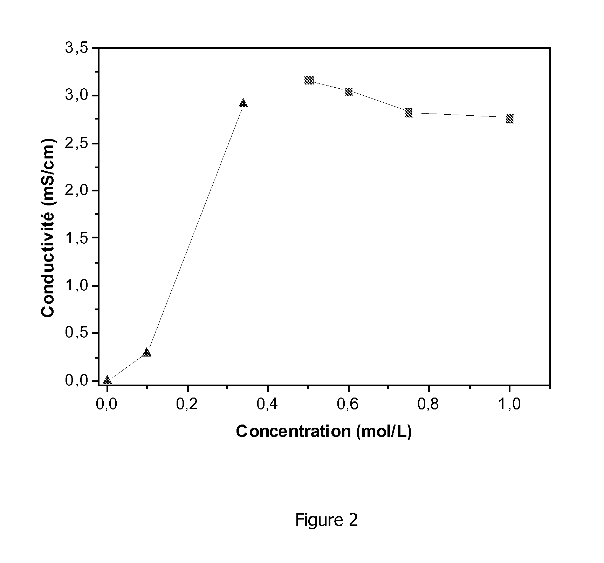

Le sel (V-1) est ensuite dissous à différentes concentrations dans un mélange d'éthylène carbone et de diméthylcarbonate avec un rapport massique de 1. La conductivité ionique des différentes concentrations est ensuite mesurée par spectroscopie d'impédance (figure 1). La stabilité électrochimique du sel (V-1) par rapport au Li+/Li est ensuite déterminée par voltamétrie cyclique d'une solution du sel (V-1) à 1 mol/L dans un mélange éthylène carbonate et diméthylcarbonate avec un rapport massique de 1 (figure 2).

## Revendications

1. Procédé de préparation des sels des composés imidazole bicycliques (V) représentés par les formules générales développées ci-dessous : 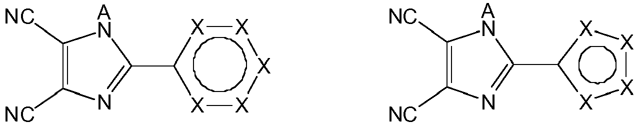 dans lesquelles A représente un cation monovalent, X représente indépendamment un atome de carbone, un atome d'oxygène, un atome de soufre, un atome de phosphore ou un atome d'azote, **caractérisé en ce que** l'on fait réagir des composés imidazole de formule (IV) représentés par les formules générales développées ci-dessous : 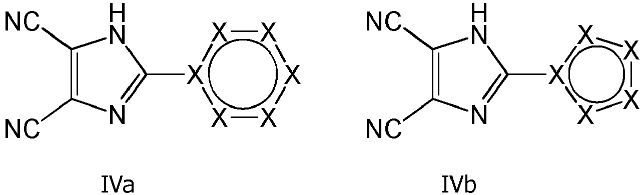 avec une base AZ, avec A ayant la même signification que ci-dessus et Z représentant un anion hydrure, hydroxyde ou carbonate.

2. Procédé de préparation des sels selon la revendication 1 **caractérisé en ce que** le cation monovalent A est un métal alcalin, de préférence choisi parmi le lithium ou le sodium.

3. Procédé de préparation des sels selon la revendication 1 ou 2 **caractérisé en ce que** X représente un atome de carbone, de phosphore ou azote.

4. Procédé de préparation des sels selon la revendication 3 **caractérisé en ce que** lesdites sels sont substitués par un groupement électro-attracteur ou électro-donneur choisis parmi un groupement cyano (CN), un groupement R₁, un groupement éther de type OR₁, un groupement amino de type N(R₁)₂, un groupement ester de type CO₂R₁, un groupement sulfonyle type SO₂R₁ ou un groupement phosphonyle type PO₂R₁, où R₁ a pour formule CₙHₘX'ₚ avec n compris entre 0 et 6, m compris entre 0 et 13, X' est un halogène (F, CI, Br et I) et p compris entre 1 et 13.

5. Procédé de préparation des sels selon la revendication 4 **caractérisé en ce que** le groupement électro-attracteur ou électro-donneur est choisi parmi l'hydrogène, le fluor, le groupement cyano (CN), le groupe trifluorométhyle (CF₃), le groupe trifluorométhoxy (OCF₃) ou le groupe méthoxy (OCH₃).

6. Procédé selon l'une quelconque des revendications 1 à 5 **caractérisé en ce que** la base AZ est choisi parmi l'hydrure de lithium, le carbonate de lithium, l'hydroxyde de lithium, l'hydrure de sodium, le carbonate de sodium, l'hydroxyde de sodium et les combinaisons de ceux-ci.

7. Procédé selon l'une quelconque des revendications 1 à 6 **caractérisé en ce que** les composés imidazole (IV) sont obtenus par condensation d'un aldéhyde aromatique de formule générale (II) représentés par les formules générales développées ci-dessous : 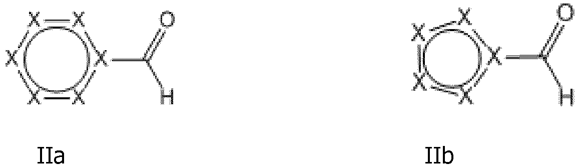 sur le diaminomaléonitrile DAMN (I) représenté par la formule : 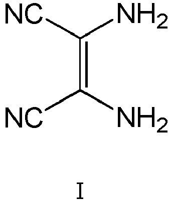 suivi d'une déshydrogénation du composé intermédiaire ainsi obtenu de formule générale (III) représenté par les formules générales développées ci-dessous :

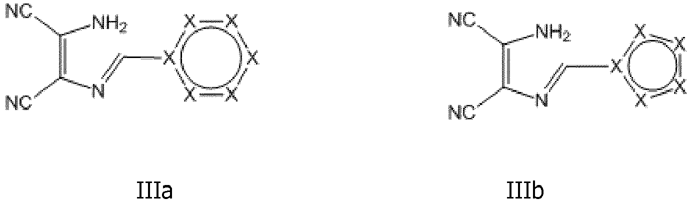

8. Procédé selon la revendication 7 comprenant (i) une étape réactionnelle du DAMN de formule (I) avec ledit aldéhyde aromatique de formule générale (II) à une température comprise entre 0 et 80°C, de préférence de 10 à 50°C, plus préférentiellement de 20 à 30°C, en présence éventuellement d'un solvant pour donner le composé de formule (III), suivie (ii) d'une étape de déshydrogénation du dit composé de formule (III).

9. Procédé selon les revendications 7 ou 8 **caractérisé en ce que** l'étape (i) est mise en oeuvre en présence d'un solvant.

10. Procédé selon la revendication 9 **caractérisé en ce que** le solvant est choisi parmi le dioxane, l'acétonitrile, ou l'ethanol.

11. Procédé selon l'une quelconque des revendications 7 à 10 **caractérisé en ce que** l'étape (i) est mise en oeuvre en présence d'un catalyseur acide.

12. Procédé selon la revendication 11 **caractérisé en ce que** le catalyseur acide est choisi parmi l'acide sulfurique, l'acide trifluoroacétique, l'acide acétique ou l'acide benzoïque.

13. Procédé selon l'une quelconque des revendications 7 à 12 **caractérisé en ce que** (ii) l'étape de déshydrogénation est mise en oeuvre en présence d'un oxydant tels que l'oxygène, l'eau oxygénée, les péroxydes, le N-chlorosuccinimide, le N-bromosuccinimide, le chlorure d'hydroxyle, le fluorure d'hydroxyle, les composés à squelette type quinone.

## Patentansprüche

1. Verfahren zur Herstellung von Salzen bicyclischer Imidazolverbindungen (V) der folgenden allgemeinen Strukturformeln: 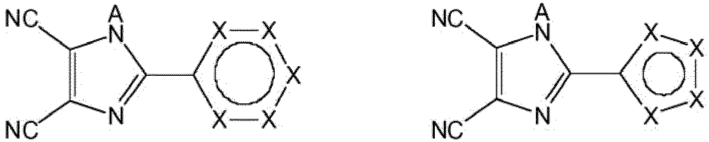 worin A ein einwertiges Kation bedeutet und die Atome X unabhängig voneinander ein Kohlenstoffatom, ein Sauerstoffatom, ein Schwefelatom, ein Phosphoratom oder ein Stickstoffatom bedeuten, **dadurch gekennzeichnet, dass** Imidazolverbindungen der Formel (IV) der folgenden allgemeinen Strukturformeln: 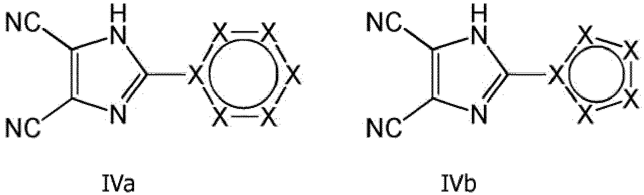 mit einer Base AZ umgesetzt werden, wobei A die oben angegebene Bedeutung hat und Z ein Hydrid-Anion, Hydroxid oder Carbonat bedeutet.

2. Verfahren zur Herstellung von Salzen nach Anspruch 1, **dadurch gekennzeichnet, dass** das einwertige Kation A ein Alkalimetall ist, das vorzugsweise unter Lithium oder Natrium ausgewählt ist.

3. Verfahren zur Herstellung von Salzen nach Anspruch 1 oder 2, **dadurch gekennzeichnet, dass** X ein Kohlenstoffatom, ein Phosphoratom oder ein Stickstoffatom bedeutet.

4. Verfahren zur Herstellung von Salzen nach Anspruch 3, **dadurch gekennzeichnet, dass** die Salze mit einer elektronenanziehenden oder elektronenliefernden Gruppe substituiert sind, die unter einer Cyanogruppe (CN), einer Gruppe R₁, einer Ethergruppe vom Typ OR₁, einer Aminogruppe vom Typ N(R₁)₂, einer Estergruppe vom Typ CO₂R₁, einer Sulfonylgruppe vom Typ SO₂R₁ oder einer Phosphonylgruppe vom Typ PO₂R₁ ausgewählt ist, wobei R₁ die Formel CₙHₘX'ₚ aufweist, worin n zwischen 0 und 6 liegt, m zwischen 0 und 13 liegt, X' ein Halogen (F, Cl, Br und I) bedeutet und p zwischen 1 und 13 liegt.

5. Verfahren zur Herstellung von Salzen nach Anspruch 4, **dadurch gekennzeichnet, dass** die elektronenanziehende oder elektronenliefernde Gruppe unter Wasserstoff, Fluor, der Cyanogruppe (CN), der Trifluormethylgruppe (CF₃), der Trifluormethoxygruppe (OCF₃) oder der Methoxygruppe (OCH₃) ausgewählt ist.

6. Verfahren nach einem der Ansprüche 1 bis 5, **dadurch gekennzeichnet, dass** die Base AZ unter Lithiumhydrid, Lithiumcarbonat, Lithiumhydroxid, Natriumhydrid, Natriumcarbonat, Natriumhydroxid und deren Kombinationen ausgewählt ist.

7. Verfahren nach einem der Ansprüche 1 bis 6, **dadurch gekennzeichnet, dass** die Imidazolverbindungen (IV) durch Kondensation eines aromatischen Aldehyden der allgemeinen Formel (II) gemäß den folgenden allgemeinen Strukturformeln: 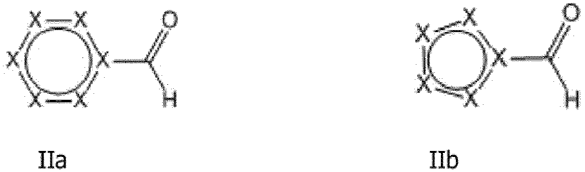 mit dem Diaminomaleonitril DAMN (I) der Formel: 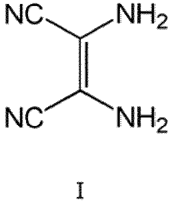 und anschließender Dehydrierung des auf diese Weise erhaltenen Zwischenprodukts der allgemeinen Formel (III) gemäß den folgenden allgemeinen Strukturformeln: 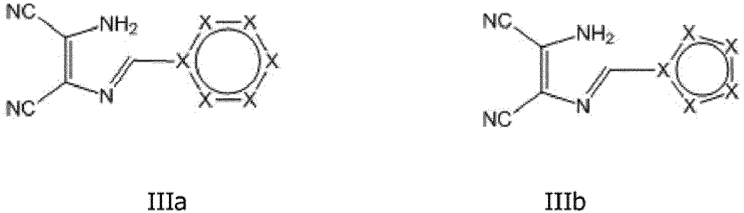 gebildet werden.

8. Verfahren nach Anspruch 7, das (i) zur Bildung der Verbindung der Formel (III) einen Schritt der Umsetzung des DAMN der Formel (I) mit dem aromatischen Aldehyden der Formel (II) bei einer Temperatur zwischen 0 und 80 °C, vorzugsweise von 10 bis 50 °C und besonders bevorzugt von 20 bis 30 °C gegebenenfalls in Gegenwart eines Lösungsmittels und anschließend (ii) einen Schritt der Dehydrierung der Verbindung der Formel (III) umfasst.

9. Verfahren nach den Ansprüchen 7 oder 8, **dadurch gekennzeichnet, dass** Schritt (i) in Gegenwart eines Lösungsmittels durchgeführt wird.

10. Verfahren nach Anspruch 9, **dadurch gekennzeichnet, dass** das Lösungsmittel unter Dioxan, Acetonitril und Ethanol ausgewählt ist.

11. Verfahren nach einem der Ansprüche 7 bis 10, **dadurch gekennzeichnet, dass** Schritt (i) in Gegenwart eines Säurekatalysators durchgeführt wird.

12. Verfahren nach Anspruch 11, **dadurch gekennzeichnet, dass** der Säurekatalysator unter Schwefelsäure, Trifluoressigsäure, Essigsäure oder Benzoesäure ausgewählt ist.

13. Verfahren nach einem der Ansprüche 7 bis 12, **dadurch gekennzeichnet, dass** Schritt (ii) der Dehydrierung in Gegenwart eines Oxidationsmittels durchgeführt wird, wie Sauerstoff, Wasserstoffperoxid, Peroxiden, N-Chlorsuccinimid, N-Bromsuccinimid, Hydroxychlorid, Hydroxyfluorid, Verbindungen mit einem Grundgerüst vom Chinontyp.

## Claims

1. A process for the preparation of a salt of the bicyclic imidazole compound (V) represented by the expanded general formulae below: 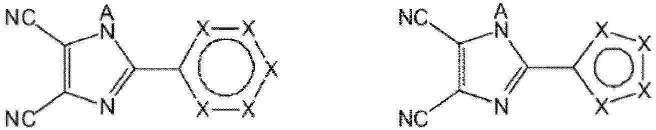 in which A represents a monovalent cation and X independently represents a carbon atom, an oxygen atom, a sulfur atom, a phosphorus atom or a nitrogen atom, **characterized in that** an imidazole compound of formula (IV) represented by the expanded general formulae below: 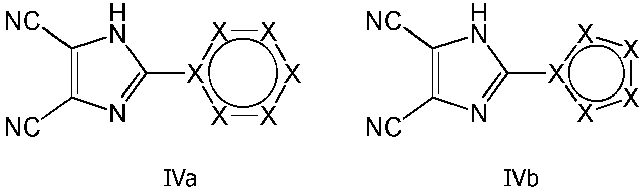 is reacted with a base AZ, with A having the same meaning as above and Z representing a hydride, hydroxide or carbonate anion.

2. The process for the preparation of a salt as claimed in claim 1, **characterized in that** the monovalent cation A is an alkali metal, preferably chosen from lithium or sodium.

3. The process for the preparation of a salt as claimed in claim 1 or 2, **characterized in that** X represents a carbon, phosphorus or nitrogen atom.

4. The process for the preparation of a salt as claimed in claim 3, **characterized in that** said salt is substituted with an electron-withdrawing or an electron-donating group preferably chosen from a cyano (CN) group, an R₁ group, an ether group of OR₁ type, an amino group of N(R₁)₂ type, an ester group of CO₂R₁ type, a sulfonyl group of SO₂R₁ type or a phosphonyl group of PO₂R₁ type, where R₁ has the formula CₙHₘX'ₚ with n between 0 and 6, m between 0 and 13, X' a halogen (F, Cl, Br and I) and p between 1 and 13.

5. The process for the preparation of a salt as claimed in claim 4, **characterized in that** the electron-withdrawing or electron-donating group is chosen from hydrogen, fluorine, the cyano (CN) group, the trifluoromethyl (CF₃) group, the trifluoromethoxy (OCF₃) group or the methoxy (OCH₃) group.

6. The process as claimed in any one of claims 1 to 5, **characterized in that** the base AZ is chosen from lithium hydride, lithium carbonate, lithium hydroxide, sodium hydride, sodium carbonate, sodium hydroxide and the combinations of these.

7. The process as claimed in any one of claims 1 to 6, **characterized in that** the imidazole compounds (IV) are obtained by condensation of an aromatic aldehyde of general formula (II) represented by the expanded general formulae below: 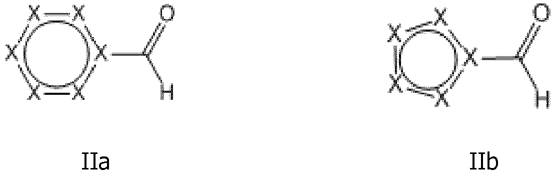 with DAMN (diaminomaleonitrile) represented by the formula (I) below: 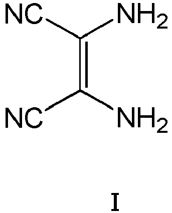 followed by dehydrogenation of the intermediate compound thus obtained of general formula (III) represented by the expanded general formulae below:

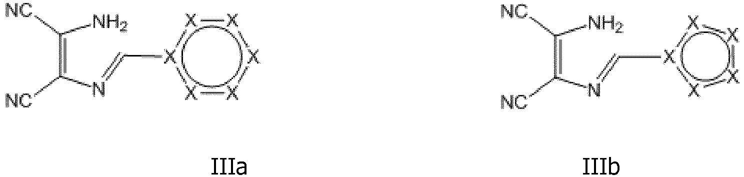

8. The process as claimed in claim 7, comprising (i) a stage of reaction of DAMN of formula (I) with an aromatic aldehyde of several formula (II) at a temperature of between 0 and 80°C, preferably from 10 to 50°C, more preferably from 20 to 30°C, optionally in the presence of a solvent, to give a compound of formula (III), followed (ii) by a stage of dehydrogenation of said compound of formula (III).

9. The process as claimed in claim 7 or claim 8, **characterized in that** stage (i) is carried out in the presence of a solvent.

10. The process as claimed in claim 9, **characterized in that** the solvent is chosen from dioxane, acetonitrile or ethanol.

11. The process as claimed in any one of claims 7 to 10, **characterized in that** stage (i) is carried out in the presence of an acid catalyst.

12. The process as claimed in claim 11, **characterized in that** the acid catalyst is chosen from sulfuric acid, trifluoroacetic acid, acetic acid or benzoic acid.

13. The process as claimed in any one of claims 7 to 12, **characterized in that** stage (ii) of dehydrogenation is carried out in the presence of an oxidizing agent, such as oxygen, hydrogen peroxide, peroxides, N-chlorosuccinimide, N-bromosuccinimide, hypochlorous acid, hypofluorous acid or compounds comprising a quinone-type backbone.
